(19) Europäisches Patentamt
European Patent Office
Office européen des brevets

(11) EP 2 611 865 B1

(12) # EUROPÄISCHE PATENTSCHRIFT

(45) Veröffentlichungstag und Bekanntmachung des Hinweises auf die Patenterteilung:
**26.12.2018 Patentblatt 2018/52**

(21) Anmeldenummer: **11738178.0**

(22) Anmeldetag: **04.07.2011**

(51) Int Cl.:
***C08L 63/00*** *(2006.01)* ***C08L 5/00*** *(2006.01)*

(86) Internationale Anmeldenummer:
**PCT/EP2011/061217**

(87) Internationale Veröffentlichungsnummer:
**WO 2012/016772 (09.02.2012 Gazette 2012/06)**

(54) **HÄRTER FÜR EPOXIDHARZE**

HARDENER FOR EPOXY RESINS

DURCISSEUR POUR RÉSINES ÉPOXY

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.08.2010 US 369943 P**

(43) Veröffentlichungstag der Anmeldung:
**10.07.2013 Patentblatt 2013/28**

(73) Patentinhaber:
- **Henkel AG & Co. KGaA**
  **40589 Düsseldorf (DE)**
- **Henkel IP & Holding GmbH**
  **40589 Düsseldorf (DE)**

(72) Erfinder:
- **WALTER, Pablo**
  **40221 Düsseldorf (DE)**
- **BENOMAR, Mustapha**
  **47137 Duisburg (DE)**
- **KREILING, Stefan**
  **69214 Eppelheim (DE)**
- **SCHÖNFELD, Rainer**
  **40627 Düsseldorf (DE)**
- **WALSH, Timothy**
  **Weymouth**
  **Massachusetts 02188 (US)**

(56) Entgegenhaltungen:
**EP-A2- 0 370 446** **EP-A2- 2 006 310**
**DE-A1- 19 729 875**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft die Verwendung von speziellen Thioetherverbindungen als Härter für reaktive Harze, Härterzusammensetzungen sowie 2- Komponentenzusammensetzungen, die diese Verbindungen enthalten, und ein Verfahren zum Verkleben von Werkstoffen, bei dem diese Verbindungen eingesetzt werden.

Reaktive Harzsysteme werden seit langem erfolgreich als Klebstoffe oder Reparaturmassen für Konsumenten, Heimwerker und Handwerker sowie in der

Luftfahrt-, Automobil- oder Elektroindustrie als Klebstoffe, Dichtstoffe oder zur Beschichtung von Oberflächen benutzt oder als Harzsysteme mit einer Reihe unterschiedlicher Materialien für die Herstellung von Verbundwerkstoffen verwendet. Härtbare Formulierungen, die Reaktivharz/Härter-Mischungen enthalten, eignen sich insbesondere als Strukturklebstoffe.

Die im Stand der Technik bekannten reaktiven Harzsysteme werden prinzipiell in zwei Klassen unterteilt. Zum einen sind 1-K-Systeme bekannt, die einen latenten Härter enthalten, der erst nach geeigneter Aktivierung, beispielsweise durch UV-Strahlung oder Wärmezufuhr, mit den anwesenden Harzen reagiert. Zum anderem sind 2-K-Systeme bekannt, bei denen der Härter bis kurz vor der eigentlichen Anwendung separat von den reaktiven Harzen konfektioniert vorliegt.

Eine bekannte Gruppe von Härtern, die üblicherweise in 2-K-Systemen eingesetzt werden, sind die so genannten Mercaptanhärter. Dabei handelt es sich in der Regel um Polysulfide und Polymercaptane mit terminalen Thiolgruppen. Da diese Härter für sich genommen nur recht langsam mit den reaktiven Harzen, insbesondere den Epoxidharzen, reagieren, werden den Härterzubereitungen häufig noch Katalysatoren zugefügt. Als derartige Katalysatoren werden häufig Amine oder Harnstoffderivate verwendet. Derartige Systeme werden umgangssprachlich häufig als 5-Minuten-Epoxidklebstoffe bezeichnet.

EP 0 370 446 A2 offenbart ein härtbares Stoffgemisch, enthaltend: a) ein Epoxidharz, b) ein Dithiol und c) ein Polyamin mit mindestens zwei primären Aminogruppen, wobei das Dithiol zwischen den beiden Thiolgruppen eine Polyethylenglykol-, eine Polypropylenglykol- oder eine Polybutylenglykolkette aufweist.

DE 197 29 875 A1 betrifft ein Härtungsmittel für Epoxidharze enthaltend eine Mercaptoverbindung, deren Moleküle mindstens fünf 1-Mercapto-2-hydroxypropylgruppen aufweisen.

EP 2 006 310 A2 offenbart eine Polymerverbindung der folgenden Formel:

$$(A^1\text{-}R^2\text{-})_n\text{-}R^1\text{-}(\text{-}P^1)_m$$

wobei $R^1$ eine organische Brückengruppe der Valenz (m+n) und $R^2$ eine Einfachbindung oder eine divalente organische Brückengruppe bedeuten. $A^1$ steht für eine monovalente organische Gruppe ausgewählt aus einer Farbstoffgruppe, einer heterocyclischen Struktur, einer Säuregruppe, einer Gruppe mit einem basischen Stickstoffatom, einer Harnstoffgruppe, einer Urethangruppe, einer Gruppe mit einem koordinierenden Sauerstoffatiom, einer Kohlenwasserstoffgruppe mit mindestens vier Kohlenstoffatomen, einer Alkoxysilylgruppe, einer Epoxygruppe, einer Isocyanatgruppe und einer Hydroxylgruppe. P1 bedeutet eine Polymerkette. m bedeutet eine Zahl von 1 bis 8, n eine Zahl von 2 bis 9 mit der Bedingung, dass die Summe (m+n) eine Zahl von 3 bis 10 ergibt.

Aufgabe der vorliegenden Erfindung war dementsprechend die Entwicklung eines Härters für reaktive Harze auf Mercaptanbasis, der auch ohne den Zusatz von Katalysatoren ein zufrieden stellendes Aushärungsverhalten aufweist und zusätzlich zu Produkten führt, die auch hinsichtlich ihrer mechanischen Eigenschaften, wie beispielsweise der Zugscherfestigkeit, den bekannten Aushärtungsprodukten auf Mercaptan/Katalysator-Basis nicht unterlegen sind.

Es wurde nun überraschenderweise gefunden, dass spezielle Thioethergruppen-haltige Verbindungen zu der erwünschten Eigenschaftskombination aus ausreichender selbstkatalysierter Härtungsgeschwindigkeit sowie Klebstoffeigenschaften führen.

Ein erster Gegenstand der vorliegenden Erfindung ist daher die Verwendung einer Thioetherverbindung, erhältlich aus einem ersten Edukt, das mindestens zwei Thiolgruppen aufweist, und einem zweiten Edukt, das mindestens eine $\alpha,\beta$-ungesättigte Amid-Gruppe sowie mindestens eine tertiäre Amin-Gruppe aufweist, zum Härten reaktiver Harze, die eine mittlere Funktionalität größer 1 aufweisen und ausgewählt sind aus Harzen auf Isocyanatbasis, Harzen auf Acrylat- und Methacrylatbasis, Anhydriden sowie Epoxid-basierten Systemen.

Erfindungsgemäß wird unter "Härter" eine Verbindung verstanden, die eine Vernetzung der reaktiven Harze bewirkt. Dementsprechend wird unter "Härten" der Prozess verstanden, der die Vernetzung der reaktiven Harze herbeiführt. Unter "Härterzubereitung" wird erfindungsgemäß das verbrauchsfertig konfektionierte Mittel verstanden. Da die erfindungsgemäß verwendeten Thioetherverbindungen in der Lage sind, die Härtungsreaktion selber zu katalysieren, kann es erfindungsgemäß bevorzugt sein, wenn die Härterzubereitung als einzigen Reaktivstoff die erfindungsgemäße Thioetherverbindung enthält.

Für den Fall, dass die erfindungsgemäß verwendete Thioetherverbindung freie Thiolgruppen aufweist, sind erfindungsgemäß auch die ganz oder teilweise deprotonierten Derivate dieser Verbindungen vom Umfang der vorliegenden Erfindung mit umfasst.

Eine wesentliche Eigenschaft der Verklebungen, die sich aus der erfindungsgemäßen Verwendung der speziellen Thioetherverbindung ergeben, ist die so genannte Zugscherfestigkeit. Diese wird mittels des folgenden Versuchsaufbaus bestimmt: Zwei sandgestrahlte, kaltgewälzte Stahlproben werden mit dem zu prüfenden Klebstoff auf einer Überlappungsfläche von 2,5 $cm^2$ mit einer Schichtdicke von 0,2mm benetzt und verklebt. Nach entsprechender Aushärtung wird die Zugscherfestigkeit des Klebstoffs gemäß DIN EN 1465 mit einer Geschwindigkeit von 15mm/min getestet. Es hat sich erfindungsgemäß als vorteilhaft erwiesen, wenn die Klebstoffe nach ihrer Aushärtung eine Zugscherfestigkeit oberhalb von 8MPa, insbesondere oberhalb von 10MPa bei Raumtemperatur aufweisen.

Ein weiterer wesentlicher Parameter der erfindungsgemäßen Verwendung ist die resultierende Verarbeitungszeit. Unter Verarbeitungszeit wird erfindungsgemäß der Zeitraum verstanden, in dem die Klebstoffzusammensetzung nach Anmischen problemlos verarbeitet werden kann, d.h. die Mischung noch Haftung zu den zu verklebenden Oberflächen aufbauen kann. Die Verarbeitungszeit wird erfindungsgemäß wie folgt bestimmt: bei 25°C werden 10g der zu prüfenden Anwendungsmischung aus den beiden Einzelkomponenten hergestellt und für 2 Minuten gründlich durchmischt. Anschließend wird die Beschaffenheit der Oberfläche der Anwendungsmischung mit Hilfe eines Holzspatels getestet, der senkrecht an die Oberfläche herangeführt wird. Die Verarbeitungszeit ist verstrichen, wenn die Anwendungsmischung nicht mehr am Holzspatel haftet, d.h. wenn sich am Holzspachtel keine Klebstofffäden mit einer Länge von mindestens 1cm mehr bilden. Erfindungsgemäß hat es sich als besonders vorteilhaft erwiesen, wenn die Verarbeitungszeit weniger als 30Minuten, vorzugsweise weniger als 10 Minuten beträgt.

Als erfindungswesentliche Komponente wird eine spezielle Thioetherverbindung verwendet, die aus einem ersten Edukt, das mindestens zwei Thiolgruppen aufweist, und einem zweiten Edukt, das mindestens eine $\alpha,\beta$-ungesättige Amid-Gruppe sowie mindestens eine tertiäre Amin-Gruppe aufweist, erhalten werden kann.

Die Bildung der erfindungsgemäßen Thioetherverbindung erfolgt vorzugsweise ohne die Zugabe von weiteren Verbindungen, wie beispielsweise Katalysatoren, bei Raumtemperatur oder geringfügig erhöhter Temperatur (ca. 20°C bis ca. 90°C). Vorzugsweise wird diese Reaktion bei ca. 70°C und einer Reaktionszeit von 2 bis 4 Stunden durchgeführt. Bezüglich einer konkreten Ausgestaltung einer derartigen Reaktion sei an dieser Stelle auf die Ausführungen im Rahmen des Beispielsteiles verwiesen.

Für die Reaktivität der erfindungsgemäß verwendeten Verbindungen spielt es keine wesentliche Rolle, ob nur ein Teil der Thiolgruppen des ersten Eduktes umgesetzt werden oder ob tatsächlich sämtliche Thiolgruppen des ersten Eduktes in Thioethergruppen überführt werden. Vielmehr sind sowohl Produkte, deren Thiolgruppen nur teilweise umgesetzt wurden, als auch Produkte, deren Thiolgruppen vollständig umgesetzt wurden, erfindungsgemäß geeignet. Selbstverständlich sind auch die Produktmischungen, wie sie sich bei derartigen Umsetzungen üblicherweise ergeben, zur Ausführung der vorliegenden Erfindung geeignet und dementsprechend mit umfasst.

Es ist erfindungsgemäß möglich, dass nur ein spezielles zweites Edukt anwesend ist, das mit den Thiolgruppen reagiert, dass heißt, dass alle Thiolgruppen während der Reaktion den gleichen Reaktionspartner antreffen. Es ist aber erfindungsgemäß auch möglich, dass mehrere voneinander verschiedene zweite Edukte angeboten werden und somit ungleichmäßig substituierte Thioetherverbindungen gebildet werden.

Ohne die Erfindung auf einen speziellen Reaktionsmechanismus beschränken zu wollen, wird davon ausgegangen, dass sich bei der genannten Umsetzung aus mindestens einer der Thiolgruppen des ersten Eduktes eine Thioethergruppe bildet. Dementsprechend sind Thioetherverbindung erfindungsgemäß bevorzugt, die

a. mindestens eine Thioethergruppe,
b. gegebenenfalls eine oder mehrere Thiol-Gruppen,
c. mindestens eine Amid-Gruppe und
d. mindestens eine tertiäre Amin-Gruppe

im Molekül aufweisen, mit der Maßgabe, dass die Thioetherverbindung mindestens zwei Schwefel-haltige funktionelle Gruppen, ausgewählt aus Thioethergruppen und/oder Thiolgruppen, aufweist.

Dabei hat es sich erfindungsgemäß als besonders vorteilhaft erwiesen, wenn die Thioetherverbindung mindestens eine Gruppe der Formel (I)

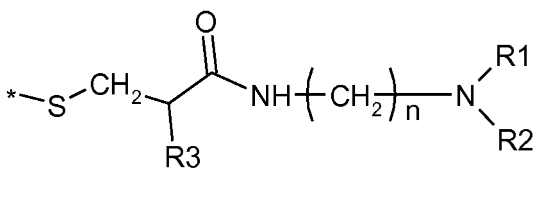

(I)

trägt, wobei

- R1 und R2 stehen unabhängig voneinander für eine verzweigte oder unverzweigte $C_1$- bis $C_{20}$-Alkylgruppe,
- R3 steht für ein Wasserstoffatom oder eine $C_1$- bis $C_4$-Alkylgruppe,
- n steht für eine ganze Zahl von 1 bis 20,
- mit der Maßgabe, dass die Gruppe der Formel (I) über ein Kohlenstoffatom an den Rest des Moleküls gebunden ist.

Beispiele für eine verzweigte oder unverzweigte $C_1$- bis $C_{20}$-Alkylgruppe sind die Gruppen Methyl, Ethyl, Propyl, Isopropyl, *n*-Butyl, *sec*.-Butyl, *tert*.-Butyl, Pentyl, Hexyl, Octyl, 2-Ethylhexyl sowie Lauryl. Erfindungsgemäß ganz besonders bevorzugt ist es, wenn die Reste R1 und R2 stehen unabhängig voneinander für eine verzweigte oder unverzweigte $C_1$- bis $C_4$-Alkylgruppe. Propyl, Ethyl und Methyl sind besonders bevorzugte $C_1$- bis $C_4$-Alkylgruppe. Eine Methylgruppe ist eine ganz besonders bevorzugte Alkylgruppe.

Thioetherverbindungen mit einer Gruppe (I) bei der R1 und/oder R2 für eine Methylgruppe stehen sind besonders bevorzugt.

Es hat sich erfindungsgemäß als vorteilhaft erwiesen, wenn die Reste R1 und R2 identische Bedeutungen haben, insbesondere wenn beide Reste R1 und R2 für eine Methylgruppe stehen.

Weiterhin ist es erfindungsgemäß bevorzugt, wenn der Rest R3 für ein Wasserstoffatom oder eine Methylgruppe steht. Reste der Formel (I), bei denen R3 für eine Methylgruppe stehen, sind ganz besonders bevorzugt.

Ferner hat es sich erfindungsgemäß als vorteilhaft erwiesen, wenn n steht für eine ganze Zahl von 1 bis 5, insbesondere für eine der Zahlen 1, 2 oder 3. Ganz besonders bevorzugt sind Reste der Formel (I), bei denen n für die Zahl 3 steht.

Ganz besonders bevorzugte Thioetherverbindungen werden erhalten, wenn Verbindungen, die zwei oder mehr Thiolgruppen aufweisen, mit Dimethylaminopropylmethacrylamid (DMAPMA; CAS: 5205-93-6) umgesetzt werden. Diese Verbindung zeichnet sich durch eine verbesserte Kompatibilität mit der Thiolverbindung, eine geringere Toxizität, ihren hohen Siedepunkt sowie ihre kommerzielle Verfügbarkeit aus.

Dabei hat sich ein Eduktverhältnis als besonders vorteilhaft erwiesen, bei dem pro Mol Thiolgruppen 0,1 bis 1 Mol Reaktionspartner zur Bildung von Thioethergruppen angeboten wird. Ein Verhältnis von 1 Mol Thiolgruppen zu 0,2 bis 1 Mol Reaktionspartner ist besonders bevorzugt.

Bei dem Thiolgruppen-haltigen Edukt kann es sich erfindungsgemäß sowohl um eine monomere Verbindung, eine oligomere Verbindung oder auch eine polymere Verbindung handeln.

Unter einem "Oligomer" wird erfindungsgemäß eine Verbindung mit weniger als 4 Wiederholungseinheiten verstanden. Dementsprechend wird unter einem "Polymer" eine Verbindung mit 4 oder mehr Wiederholungseinheiten verstanden.

Dementsprechend ist es erfindungsgemäß bevorzugt, wenn die Thioetherverbindung eine Verbindung der Formel (II)

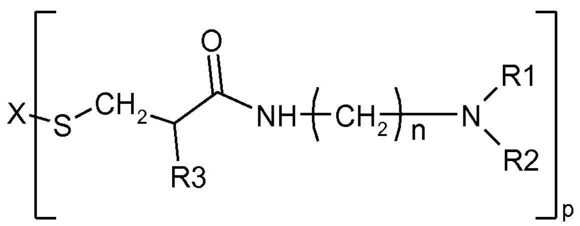

(II)

ist, wobei

- R1 und R2 stehen unabhängig voneinander für eine verzweigte oder unverzweigte $C_1$- bis $C_{20}$-Alkylgruppe,
- R3 steht für ein Wasserstoffatom oder eine $C_1$- bis $C_4$-Alkylgruppe,
- n steht für eine ganze Zahl von 1 bis 20,
- p steht für eine rationale Zahl von 1,0 bis 5,0 und
- X steht für einen p-valenten Rest, der jeweils über ein Kohlenstoffatom an die funktionelle Gruppe der Formel (I) gebunden ist, und gegebenenfalls mindestens eine Thiol-Gruppe und/oder mindestens eine Thioether-Gruppe aufweist,
- mit der Maßgabe, dass X zwingend mindestens eine Thiol-Gruppe und/oder mindestens eine Thioether-Gruppe aufweist, wenn p=1 ist.

Bezüglich der besonders bevorzugten Ausführungsformen der Reste R1, R2 und R3 sowie der Zahl n sei an dieser Stelle explizit auf die obigen Ausführungen verwiesen.

Der Rest X steht erfindungsgemäß bevorzugt für einen oligomeren oder polymeren Rest.

Weiterhin ist es bevorzugt, wenn der Rest X Alkylendioleinheiten, insbesondere Ethylenglykoleinheiten und/oder Propylenglykoleinheiten und/oder Butandioleinheiten aufweist.

Reste X, die zwei oder mehr Alkylendioleinheiten aufweisen, sind erfindungsgemäß ganz besonders bevorzugt. Insbesondere Reste X, die zwei oder mehr Ethylenglykoleinheiten und/oder zwei oder mehr, insbesondere drei, Propylenglykoleinheiten enthalten sind besonders bevorzugt.

Da die erfindungsgemäß bevorzugten Thioetherverbindungen unabhängig von dem gewählten Syntheseweg eine gute Wirksamkeit als Härter für reaktive Harze aufweisen, sind erfindungsgemäß auch Verbindungen der Formel (II) umfasst, die aus anderen Edukten als oben beschrieben erhalten werden können.

Eine erste Gruppe erfindungsgemäß erster Edukte, die zwei oder mehr Thiolgruppen aufweisen und nach Reaktion die erfindungsgemäßen Verbindungen der Formel (II) ergeben, basiert auf alkoxylierten Derivaten des Glycerins. Erfindungsgemäß sind Glycerinderivate mit einem Alkoxylierungsgrad von 2 bis 9 besonders bevorzugt. Ein Alkoxylierungsgrad von 2 bis 5 ist ganz besonders bevorzugt. Auch in dieser Gruppe sind Propylenglykol und Ethylenglykol bevorzugte Alkylendiole. Propylenglykol ist in dieser Ausführungsform ganz besonders bevorzugt.

Ein besonders bevorzugtes Edukt dieser Gruppe ist:

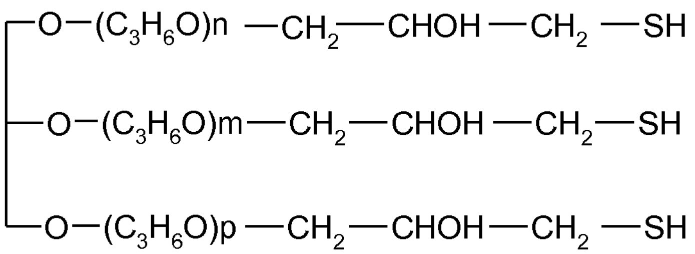

(III)

wobei n, m und p stehen unabhängig voneinander jeweils für eine ganze Zahl von 0 bis 3, mit der Maßgabe, dass die Summe n+m+p für eine ganze Zahl von 2 bis 9, insbesondere für eine ganze Zahl von 2 bis 5, steht.

Besonders bevorzugt sind erfindungsgemäß Thioetherverbindungen, die als Reaktionsprodukt einer Umsetzung einer Verbindung der Formel (III) mit Dimethylaminopropylmethacrylamid (DMAPMA) erhalten werden. Dabei hat sich ein Eduktverhältnis als besonders vorteilhaft erwiesen, bei dem pro Mol Thiolgruppen 0,1 bis 1 Mol DMAPMA zur Bildung von Thioethergruppen angeboten wird. Ein Verhältnis von 1 Mol Thiolgruppen zu 0,2 bis 1 Mol DMAPMA ist besonders bevorzugt.

Eine zweite Gruppe derartiger Edukte leitet sich von den Polyalkylendiolen ab. Im Rahmen dieser Gruppe sind Polyalkylendiole mit 2 bis 5 Alkylendiol-Einheiten besonders bevorzugt. Auch in dieser Gruppe sind Propylenglykol und Ethylenglykol bevorzugte Alkylenglykole. Ethylenglykol ist in dieser Ausführungsform ganz besonders bevorzugt.

Ein besonders bevorzugtes Edukt dieser Gruppe ist

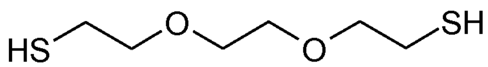

(1,8-Dimercapto-3,6-Dioxaoctan; DMDO).

Ebenfalls erfindungsgemäß geeignete Edukte sind Präpolymere, die aus DMDO gewonnen werden können.

Besonders bevorzugte sind erfindungsgemäß Thioetherverbindungen, die als Reaktionsprodukt einer Umsetzung von 1,8-Dimercapto-3,6-Dioxaoctan und/oder einem Präpolymeren daraus mit Dimethylaminopropylmethacrylamid (DMAPMA) erhalten werden. Dabei hat sich ein Eduktverhältnis als besonders vorteilhaft erwiesen, bei dem pro Mol Thiolgruppen 0,1 bis 1 Mol DMAPMA zur Bildung von Thioethergruppen angeboten wird. Ein Verhältnis von 1 Mol Thiolgruppen zu 0,2 bis 1 Mol DMAPMA ist besonders bevorzugt.

Die hier beschriebenen Thioetherverbindungen werden erfindungsgemäß zur Härtung reaktiver Harze verwendet.

Erfindungsgemäß wird unter einem "reaktiven Harz" eine Verbindung verstanden, die eine mittlere Funktionalität größer 1 aufweist. Aufgrund der reaktiven Gruppen kann die Verbindung mit geeigneten Härtern umgesetzt und hierdurch gehärtet werden, sofern eine geeignete Reaktivität vorliegt. Diese ist bei den Harzen auf Isocyanatbasis, Harzen auf Acrylat- und Methacrylatbasis, Anhydriden sowie den Epoxid-basierten Systemen gegeben. Es hat sich erfindungsgemäß als besonders vorteilhaft erwiesen, wenn die Thioetherverbindungen zum Härten von Epoxidharzen eingesetzt werden.

Im Rahmen der vorliegenden Erfindung erfindungsgemäß härtbare Epoxidharze sind vorzugsweise ausgewählt aus Epoxidharze vom Bisphenol-A-Typ, Epoxidharze vom BisphenolS-Typ, Epoxidharze vom Bisphenol-F-Typ, Epoxidharze vom Phenol-Novolak-Typ, Epoxidharze vom Cresol-Novolak-Typ, epoxidierte Produkte zahlreicher Dicyclopentadien-modifizierter Phenolharze, erhältlich durch Umsetzung von Dicyclopentadien mit zahlreichen Phenolen, epoxidierte Produkte von 2,2',6,6'-Tetramethylbiphenol, aromatische Epoxidharze wie Epoxidharze mit Naphthalin-Grundgerüst und Epoxidharze mit Fluoren-Grundgerüst, aliphatische Epoxidharze wie Neopentylglykoldiglycidylether und 1,6-Hexandioldiglycidylether, alicyclische Epoxidharze wie 3,4-Epoxycyclohexylmethyl-3,4-epoxycyclohexancarboxylat und Bis(3,4-epoxycyclohexyl)adipat, und Epoxidharze mit einem Heteroring wie Triglycidylisocyanurat ein.

Besonders bevorzugte Epoxidharze sind die Reaktionsprodukte aus Bisphenol A und Epichlorhydrin, die Reaktionsprodukte aus Phenol und Formaldehyd (Novolak-Harze) und Epichlorhydrin, Glycidyl Ester sowie die Reaktionsprodukt aus Epichlorhydrin und p-Aminophenol.

Weitere bevorzugte Epoxidharze, die kommerziell erhältlich sind, sind insbesondere Octadecylenoxid, Epichlorhydrin, Styroloxid, Vinylcyclohexenoxid, Glycidol, Glycidylmethacrylat, Diglycidylether von Bisphenol A (beispielsweise diejenigen, welche unter den Handelsbezeichnungen "Epon 828", "Epon 825", "Epon 1004"; "Epon 1007", "Epon 1002", "Epon 1001" und "Epon 1010" der Hexion Specialty Chemicals Inc., "DER-331", "DER-332", "DER-334", "DER-354", "DER-732" und "DER-736" der Dow Chemical Co. erhältlich sind), Vinylcyclohexendioxid, 3,4-Epoxycyclohexylmethyl-3,4-epoxycyclohexen-carboxylat, 3,4-Epoxy-6-methylcyclohexylmethyl-3,4-epoxy-6-methyl-cyclohexencarboxylat, Bis(3,4-epoxy-6-methylcyclohexylmethyl)adipat, Bis(2,3-epoxy-cyclopentyl)ether, aliphatisches, mit Polypropylenglycol modifiziertes Epoxid, Dipentendioxid, epoxidiertes Polybutadien (beispielsweise Krasol Produkte von Sartomer), Epoxidfunktionalitaet enthaltendes Siliconharz, flammhemmende Epoxidharze (beispielsweise "DER-580", ein bromiertes Epoxidharz vom Bisphenol-Typ, welches von Dow Chemical Co. erhaeltlich ist), 1,4-Butandiol-diglycidylether eines Phenolformaldehyd-Novolaks (beispielsweise "DEN-431" und "DEN-438" der Dow Chemical Co.), sowie Resorcin-diglycidylether (beispielsweise "Kopoxite" der Koppers Company Inc.), Bis(3,4-epoxycyclohexyl)adipat, 2-(3,4-Epoxycyclohexyl-5,5-spiro-3,4-epoxy)cyclohexan-meta-dioxan, Vinylcyclohexenmonoxid, 1,2-Epoxyhexadecan, Alkylglycidilether wie beispielsweise $C_8$-$C_{10}$-Alkyl-glycidylether (beispielsweise "HELOXY Modifier 7" der Hexion Specialty Chemicals Inc.), $C_{12}$-$C_{14}$-Alkyl-glycidylether (beispielsweise. "HELOXY Modifier 8" der Hexion Specialty Chemicals Inc.), Butylglycidylether (beispielsweise "HELOXY Modifier 61" der Hexion Specialty Chemicals Inc.), Cresylglycidylether (beispielsweise "HELOXY Modifier 62" der Hexion Specialty Chemicals Inc.), p-tert.-Butylphenyl-glycidylether (beispielsweise "HELOXY Modifier 65" der Hexion Specialty Chemicals Inc.), polyfunktionelle Glycidylether wie beispielsweise. Diglycidylether von 1,4-Butandiol (beispielsweise "HELOXY Modifier 67" der Hexion Specialty Chemicals Inc.), Diglycidylether von Neopentylglycol (beispielsweise "HELOXY Modifier 68" der Hexion Specialty Chemicals Inc.), Diglycidylether von Cyclohexandimethanol (beispielsweise "HELOXY Modifier 107" der Hexion Specialty Chemicals Inc.), Trimethylolethantriglycidilether (beispielsweise. "HELOXY Modifier 44" der Hexion Specialty Chemicals Inc.), Trimethylolpropan-triglycidylether (beispielsweise "HELOXY Modifier 48" der Hexion Specialty Chemicals Inc.), Polyglycidylether eines aliphatischen Polyols (beispielsweise "HELOXY Modifier 84" der Hexion Specialty Chemicals Inc.), Polyglycoldiepoxid (beispielsweise "HELOXY Modifier 32" der Hexion Specialty Chemicals Inc.), Bisphenol F-Epoxide (beispielsweise "EPN-1138" oder GY-281" der Huntsman Int. LLC), 9,9-Bis-4-(2,3-epoxypropoxy)-phenylfluorenon (beispielsweise "Epon 1079" der Hexion Specialty Chemicals Inc.).

Weitere bevorzugte, kommerziell erhältliche Verbindungen sind beispielsweise ausgewählt aus AralditeTM 6010, AralditTM GY-281TM, AralditTM ECN-1273, AralditTM ECN-1280, AralditTM MY-720, RD-2 existiert von der Huntsman Int. LLC; DENTM 432, DENTM 438, DENTM 485 von Dow Chemical Co., EponTM 812, 826, 830, 834, 836, 871, 872,1001, 1031 etc. von Hexion Specialty Chemicals Inc. und HPTTM 1071, HPTTM 1079 ebenfalls von Hexion Specialty Chemicals Inc., als Novolak-Harze weiterhin beispielsweise Epi-RezTM 5132 von Hexion Specialty Chemicals Inc., ESCN-001 von Sumitomo Chemical, Quatrex 5010 von Dow Chemical Co., RE 305S von Nippon Kayaku, EpiclonTM N673 von DaiNipon Ink Chemistry oder EpicoteTM 152 von Hexion Specialty Chemicals Inc..

Weitere Epoxidharze können vorzugsweise Copolymere von Acrylsäureestern mit Glycidol wie beispielsweise Glycidylacrylat und Glycidylmethacrylat mit einer oder mehreren copolymerisierbaren Vinylverbindungen sein. Beispiele solcher Copolymeren sind 1:1 Styrol-Glycidylmethacrylat, 1:1 Methylmethacrylat-Glycidylacrylat und 62.5:24:13.5 Methylmethacrylat-Ethylacrylat-Glycidylmethacrylat enthalten.

Weitere verwendbare Epoxidharze sind Silicone mit Epoxidfunktionalität, insbesondere Cyclohexylepoxid-Gruppen, insbesondere solche mit einem Silicon-Grundgerüst. Beispiele sind UV 9300, UV 9315, UV 9400 und UV 9425, welche alle von GE Bayer Silicones geliefert werden.

In einer bevorzugten Ausführungsform umfassen die erfindungsgemäßen Zubereitungen eine Mischung aus mehreren der genannten Epoxidharze.

Beispiele solcher Mischungen können zwei oder mehrere Molekulargewichtsverteilungen von epoxidhaltigen Verbindungen, wie beispielsweise ein niedriges Molekulargewicht (unter 200), ein mittleres Molekulargewicht (ca. 200 bis 10000) und ein höheres Molekulargewicht (über ca. 10000) umfassen. Alternativ oder zusätzlich kann das Epoxidharz eine Mischung von epoxidhaltigen Materialien unterschiedlicher chemischer Natur (beispielsweise aliphatisch oder aromatisch) oder Funktionalität (beispielsweise polar oder unpolar) enthalten.

Ein zweiter Gegenstand der vorliegenden Erfindung ist eine Härterzusammensetzung für reaktive Harze, die mindestens eine erfindungsgemäße Thioetherverbindung sowie einen Beschleuniger, der mindestens ein Stickstoffatom aufweist, enthält, wobei die reaktiven Harze eine mittlere Funktionalität größer 1 aufweisen und ausgewählt sind aus Harzen auf Isocyanatbasis, Harzen auf Acrylat- und Methacrylatbasis, Anhydriden sowie Epoxid-basierten Systemen.

Obwohl die erfindungsgemäßen Thioetherverbindungen sich dadurch auszeichnen, dass sie eine akzeptable Reaktionszeit auch ohne den Zusatz einer Beschleunigerkomponente aufweisen (Autokatalyse), kann es erfindungsgemäß bevorzugt sein, wenn ein Beschleuniger bei der Reaktion anwesend ist.

Bevorzugte Beispiele für derartige Beschleuniger sind katalytisch wirksame substituierte Harnstoffe. Dies sind insbesondere der N,N'-(Di-(3-Dimethylaminopropyl))harnstoff. Prinzipiell können auch katalytisch wirksame tertiäre Acryl- oder Alkyl-Amine, wie beispielsweise das Benzyldimethylamin, Tris(dimethylaminomethyl)phenol, Piperidin oder Piperidinderivate eingesetzt werden. Weiterhin können diverse, vorzugsweise feste Imidazolderivate als katalytisch wirksame Beschleuniger eingesetzt werden. Stellvertretend genannt seien 2-Ethyl-2-methylimidazol, N-Butylimidazol, Benzimidazol sowie N-$C_1$ bis $C_{12}$-Alkylimidazole oder N-Arylimidazole. Weiterhin eigenen sich Addukte von Aminoverbindungen an Epoxidharze als beschleunigende Zusätze zu den vorgenannten Härtern. Geeignete Aminoverbindungen sind tertiäre aliphatische, aromatische oder cyclische Amine. Geeignete Epoxyverbindungen sind beispielsweise Polyepoxide auf Basis von Glycidylethern des Bisphenols A oder F oder des Resorcins. Konkrete Beispiele für solche Addukte sind Addukte von tertiären Aminen wie 2-Dimethylaminoethanol, N-substituierte Piperazine, N-substituierte Homopiperazine, N-substituierte Aminophenole an Di- oder Polyglycidylether des Bisphenols A oder F oder des Resorcins.

Erfindungsgemäß ganz besonders bevorzugte Beschleuniger sind 2-Dimethylaminoethanol, Tris(dimethylaminomethyl)phenol und N,N'-(Di-(3-Dimethylaminopropyl))harnstoff. Der zuletzt genannte Beschleuniger ist beispielsweise unter den Handelnamen Capcure® EH 50 und Versamine® EH 50 kommerziell erhältlich.

Ein dritter Gegenstand der vorliegenden Erfindung sind zweikomponentige Zusammensetzungen, deren erste Zubereitung mindestens ein reaktives Harz enthält, das eine mittlere Funktionalität größer 1 aufweist und ausgewählt ist aus Harzen auf Isocyanatbasis, Harzen auf Acrylat- und Methacrylatbasis, Anhydriden sowie Epoxid-basierten Systemen, und deren zweite Zubereitung mindestens eine erfindungsgemäße Thioetherverbindung enthält.

Hinsichtlich der im Rahmen dieses Gegenstandes der vorliegenden Erfindung besonders geeigneten Harze und Thioetherverbindungen sei an dieser Stelle explizit auf die obigen Ausführungen verwiesen.

In einer bevorzugten Ausführungsform dieses Gegenstandes der vorliegenden Erfindung enthält die erste Zubereitung der Zweikomponentenzusammensetzung als reaktiven Harz eine Mischung aus

a) mindestens einem Epoxidgruppen-haltigen Reaktionsprodukts von Epichlorhydrin mit Polypropylenglykol, das ein Epoxid-Äquivalentgewicht von mindestens 250g/eq aufweist, sowie

b) mindestens einem Epoxidgruppen-haltigen Reaktionsprodukts von Epichlorhydrin mit einem Novolakharz, das ein Epoxid-Äquivalentgewicht von mindestens 175g/eq aufweist, enthält.

In einer bevorzugten Ausführungsform enthält die erste Zubereitung 10 bis 60 Gew.-% der Komponente a). Ganz bevorzugt ist ein Mengenbereich von 25 bis 45 Gew.-%. Die Mengenangaben beziehen sich dabei jeweils auf die Mischung aller reaktiven Epoxidharze ohne die weiteren Formulierungsbestandteile.

Im Rahmen der dieser Erfindung zu Grunde liegenden Arbeiten konnte gezeigt werden, dass Epoxidgruppenhaltige Reaktionsprodukte von Epichlorhydrin mit Polypropylenglykol mit einem Epoxid-Äquivalentgewicht von mindestens 300g/eq besonders vorteilhafte Eigenschaften aufweisen.

Besonders bevorzugte Komponenten a) sind erfindungsgemäß die unter den Handelsbezeichnungen DER 732 (EEW 310-330 g/eq), DER 732P (EEW 310-330 g/eq), von der Firma Dow vertriebenen reaktiven Epoxidharze.

Besonders vorteilhaft ist es erfindungsgemäß, wenn entsprechende Epoxidgruppen-haltige Reaktionsprodukte von Epichlorhydrin mit Polypropylenglykol mit einem Epoxid-Äquivalentgewicht von weniger als 300g/eq maximal zu einem Anteil von 3 Gew.-%, insbesondere von maximal 1 Gew.-%, jeweils bezogen auf die gesamte Zusammensetzung, enthalten sind.

In einer bevorzugten Ausführungsform enthält die erste Zubereitung der erfindungsgemäßen Zweikomponentenzusammensetzung 10 bis 85 Gew.-% der Komponente b). Ganz bevorzugt ist ein Mengenbereich von 30 bis 75Gew.-%. Die Mengenangaben beziehen sich dabei jeweils auf die Mischung aller Epoxidharze ohne die weiteren Formulierungsbestandteile.

Im Rahmen der dieser Erfindung zu Grunde liegenden Arbeiten konnte gezeigt werden, dass Epoxidgruppenhaltige Reaktionsprodukte von Epichlorhydrin mit einem Novolak mit einem Epoxid-Äquivalentgewicht von mindestens 180g/eq, und insbesondere von mindestens 190g/eq, besonders vorteilhafte Eigenschaften aufweisen.

Erfindungsgemäß bevorzugte Novolake sind die Polykondensationsprodukte von Formaldehyd mit Phenol und/oder Cresol.

Darüber hinaus hat es sich als vorteilhaft erwiesen, wenn die Reaktionsprodukte aus Epichlorhydrin und Novolak eine Epoxidfunktionaltität von mindestens 3, insbesondere von mindestens 3,5 aufweist.

Reaktionsprodukte aus Epichlorhydrin und Novolak, die ein Epoxid-Äquivalentgewicht von mindestens 175g/eq und gleichzeitig eine Epoxidfunktionaltität von mindestens 3 aufweisen, konnten hinsichtlich der erfindungsgemäß gestellten Aufgaben besonders überzeugen. Besonders bevorzugt sind Reaktionsprodukte von Epichlorhydrin und Novolak

mit einem Epoxid-Äquivalentgewicht von mindestens 180g/eq und einer Epoxidfunktionaltität von mindestens 3, insbesondere Reaktionsprodukte von Epichlorhydrin und Novolak mit einem Epoxid-Äquivalentgewicht von mindestens 190g/eq und einer Eopxidfunktionaltität von mindestens 3,5.

Hierdurch wird am besten die erwünschte Eigenschaftskombination von Verarbeitbarkeit vor dem Aushärten und Klebefestigkeit nach dem Aushärten erreicht.

Besonders bevorzugte Komponenten b) sind erfindungsgemäß die unter den Handelsbezeichnungen DEN 439 (EEW 191-210g/eq; Funktionalität 3,8; Dow), Araldite ECN 1299 (Cresol-Formaldehyd-Novolak; EEW 235 g/eq; Funktionalität 2,5-5,5; Huntsman), Epikote 154 (EEW 176-181 g/eq; Hexion) vertriebenen reaktiven Epoxyharze. DEN 439 ist erfindungsgemäß ganz besonders bevorzugt.

Besonders vorteilhaft ist es erfindungsgemäß, wenn entsprechende Reaktionsprodukte von Epichlorhydrin und Novolak mit einem Epoxid-Äquivalentgewicht von weniger als 175g/eq maximal zu einem Anteil von 3 Gew.-%, insbesondere maximal zu einem Anteil von 1 Gew.-%, jeweils bezogen auf die gesamte Zusammensetzung, enthalten sind.

In einer bevorzugten Ausführungsform der vorliegenden Erfindung enthält die Epoxidharzmischung weiterhin mindestens ein Epoxidgruppen-haltiges Reaktionsprodukt von Epichlorhydrin mit Bisphenol A, das ein Epoxid-Äquivalentgewicht von mindestens 500g/eq aufweist.

In dieser Ausführungsform sind dementsprechend erste Zubereitungen besonders bevorzugt, die bezogen auf die Masse aller Epoxidharze, die folgenden Komponenten enthalten:

H a) 10 - 60Gew.-% eines Epoxidgruppen-haltigen Reaktionsprodukts von Epichlorhydrin mit Polypropylenglykol, das ein Epoxid-Äquivalentgewicht von mindestens 250g/eq aufweist,
H b) 15 - 85Gew.-% eines Epoxidgruppen-haltigen Reaktionsprodukts von Epichlorhydrin mit einem Novolakharz, das ein Epoxid-Äquivalentgewicht von mindestens 175g/eq aufweist, sowie
H c) 0 - 70Gew.-% eines Epoxidgruppen-haltigen Reaktionsprodukts von Epichlorhydrin mit mindestens einem Bisphenol, das ein Epoxid-Äquivalentgewicht von mindestens 500g/eq aufweist.

Besonders bevorzugt sind erfindungsgemäß Zusammensetzungen, die bezogen auf die Masse aller Epoxidharze, die folgenden Komponenten enthalten:

H a) 30 - 45Gew.-% eines Epoxidgruppen-haltigen Reaktionsprodukts von Epichlorhydrin mit Polypropylenglykol, das ein Epoxid-Äquivalentgewicht von mindestens 250g/eq aufweist,
H b) 30- 45Gew.-% eines Epoxidgruppen-haltigen Reaktionsprodukts von Epichlorhydrin mit einem Novolakharz, das ein Epoxid-Äquivalentgewicht von mindestens 175g/eq aufweist, sowie
H c) 10 - 40Gew.-% eines Epoxidgruppen-haltigen Reaktionsprodukts von Epichlorhydrin mit mindestens einem Bisphenol, das ein Epoxid-Äquivalentgewicht von mindestens 500g/eq aufweist.

Im Rahmen der dieser Erfindung zu Grunde liegenden Arbeiten konnte gezeigt werden, dass Epoxidgruppenhaltigen Reaktionsprodukte von Epichlorhydrin mit Bisphenol A, das ein Epoxid-Äquivalentgewicht von mindestens 560g/eq besonders vorteilhafte Eigenschaften aufweisen.

Erfindungsgemäß besonders bevorzugte Epoxidgruppen-haltigen Reaktionsprodukte von Epichlorhydrin mit Bisphenol A, das ein Epoxid-Äquivalentgewicht von mindestens 500g/eq sind unter den Handelsbezeichnungen

H Epikote® 1002 (EEW 575-700g/eq; Epoxy-Funtionalität 2; Hexion)
H DER® 662E (EEW 590-630g/eq; Epoxy-Funktionalität 2)
H Epon® 1002F (EEW 600-700g/eq; Epoxy-Funktionalität 2)
H DER® 662UH (EEW 675-750g/eq; Epoxy-Funktionalität 2)
H DER® 663U (EEW 730-820g/eq; Epoxy-Funktionalität 2)
H DER® 664U (EEW 875-955g/eq; Epoxy-Funktionalität 2)
H Epon® 1009F (EEW 2300 - 3800g/eq; Epoxy-Funktionalität 2; Hexion)
H Epon® 1007F (EEW 1700-2300g/eq; Epoxyfunktionalität 2; Hexion)
H Epon® 1004F (EEW 800-950g/eq; Epoxy-Funktionalität 2; Hexion)
H DER® 692H (EEW 660-720g/eq; Eopxy-Funktionalität 2; Dow)
H DER® 692 (EEW 660-720g/eq; Epoxy-Funktionalität 2; Dow)

vertriebenen Epoxyharze. Die unter den Handelsbezeichnungen Epikote® 1002, DER® 662E und Epon® 1002F vertriebenen Produkte sind erfindungsgemäß ganz besonders bevorzugt. Epon® 1002F ist erfindungsgemäß ganz besonders bevorzugt.

Besonders vorteilhaft ist es erfindungsgemäß, wenn entsprechende Epoxidgruppen-haltige Reaktionsprodukte von Epichlorhydrin mit Bisphenol A, die ein Epoxid-Äquivalentgewicht unterhalb von 500g/eq aufweisen, maximal zu

einem Anteil von 3 Gew.-%, insbesondere maximal zu einem Anteil von 1 Gew.-%, jeweils bezogen auf die gesamte Zusammensetzung, enthalten sind.

Insbesondere in toxikologischer Hinsicht hat es sich als vorteilhaft erwiesen, wenn die erfindungsgemäßen Zusammensetzungen neben den genannten erfindungswesentlichen Epoxidgruppen-haltigen Reaktionsprodukten a), b) und c) weniger als 3 Gew.-%, insbesondere weniger als 1 Gew.-% weiterer Epoxidgruppen-haltiger Reaktionsprodukte enthalten, jeweils bezogen auf die gesamte Zubereitung.

In einer besonders bevorzugten Ausführungsform dieses Gegenstandes besteht die Mischung der Epoxidharze aus

- a) 10 - 60Gew.-% eines Epoxidgruppen-haltigen Reaktionsprodukts von Epichlorhydrin mit Polypropylenglykol, das ein Epoxid-Äquivalentgewicht von mindestens 250g/eq aufweist,
- b) 15 - 85Gew.-% eines Epoxidgruppen-haltigen Reaktionsprodukts von Epichlorhydrin mit einem Novolakharz, das ein Epoxid-Äquivalentgewicht von mindestens 175g/eq aufweist, sowie
- c) 0 - 70Gew.-% eines Epoxidgruppen-haltigen Reaktionsprodukts von Epichlorhydrin mit mindestens einem Bisphenol, das ein Epoxid-Äquivalentgewicht von mindestens 500g/eq aufweist,

wobei sich die Mengen der Bestandteile a), b) und c) zu 100Gew.-% ergänzen.

In einer ganz besonders bevorzugten Ausführungsform dieses Gegenstandes besteht die Mischung der Epoxidharze aus

- a) 30 - 45Gew.-% eines Epoxidgruppen-haltigen Reaktionsprodukts von Epichlorhydrin mit Polypropylenglykol, das ein Epoxid-Äquivalentgewicht von mindestens 250g/eq aufweist,
- b) 30- 45Gew.-% eines Epoxidgruppen-haltigen Reaktionsprodukts von Epichlorhydrin mit einem Novolakharz, das ein Epoxid-Äquivalentgewicht von mindestens 175g/eq aufweist, sowie
- c) 10 - 40Gew.-% eines Epoxidgruppen-haltigen Reaktionsprodukts von Epichlorhydrin mit mindestens einem Bisphenol, das ein Epoxid-Äquivalentgewicht von mindestens 500g/eq aufweist,

wobei sich die Mengen der Bestandteile a), b) und c) zu 100Gew.-% ergänzen.

Die Thioetherverbindungen werden als Härter vorzugsweise in einem Verhältnis von 1 Thiol/Thioether Equivalent Härter pro 1 - 3 Epoxy Equivalent. eingesetzt. Dies bedeutet, dass die Anwendungsmischung vorzugsweise einen Überschuss an reaktiven Epoxygruppen gegenüber den reaktiven Thiol- und Thioethergruppen aufweist.

Zur Verbesserung des Bruchverhaltens, insbesondere bei Temperaturen unterhalb von 0 °C, können die erfindungsgemäßen Zweikomponenten-Zubereitungen einen oder mehrere unterschiedliche so genannte Schlagzähigkeits-Verbesserer (englisch: "toughener") enthalten. Derartige Schlagzähigkeits-Verbesserer sind dem Fachmann auf dem Gebiet der EpoxidKlebstoffe bekannt. Beispielsweise können sie ausgewählt sein aus: Thermoplastischen Isocyanaten oder Polyurethanen, Kautschuk-Partikeln, insbesondere solchen mit Kern-Schale-Struktur, und Block-Copolymeren, insbesondere solchen, die einen ersten Polymerblock mit einer Glasübergangstemperatur von unterhalb 15 °C und einen zweiten Polymerblock mit einer Glasübergangstemperatur von oberhalb 25 °C enthalten. Derartige Block-Copolymere sind vorzugsweise ausgewählt aus solchen, bei denen ein erster Polymerblock ausgewählt ist aus einem Polybutadien- oder Polyisoprenblock und ein zweiter Polymerblock ausgewählt ist aus einem Polystyrol- oder einem Polymethylmethacrylat-Block. Spezielle Beispiele hierfür sind Block-Copolymere mit folgendem Blockaufbau: Styrol-Butadien-(Meth)Acrylat, Styrol-Butadien-(Meth)Acrylsäureester, Ethylen-(Meth)Acrylsäureester-Glycidyl(meth)acrylsäureester, Ethylen-(Meth)Acrylsäureester-Maleinsäureanhydrid, Methylmethacrylat-Butylacrylat-Methylmethacrylat.

Weiterhin hat es sich als erfindungsgemäß vorteilhaft erwiesen, wenn die erfindungsgemäßen Zweikomponenten-Zusammensetzungen neben der Mischung von Epoxidharzen und dem erfindungsgemäßen Härter mindestens einen anorganischen und/oder organischen Füllstoff enthält.

Erfindungsgemäß bevorzugt Füllstoffe sind zum Beispiel die diversen gemahlenen oder gefällten Kreiden, Ruß, Calcium-Magnesiumcarbonate, Talkum, Schwerspat sowie insbesondere silicatische Füllstoffe vom Typ des Aluminium-Magnesium-Calcium-Silicats, z. B. Wollastonit, Chlorit.

Zur Gewichtsreduzierung kann die Zubereitung zusätzlich zu den vorgenannten "normalen" Füllstoffen so genannte Leichtfüllstoffe enthalten. Diese können ausgewählt werden aus der Gruppe der Metallhohlkugeln wie beispielsweise Stahlhohlkugeln, Glashohlkugeln, Flugasche (Fillite), Kunststoffhohlkugeln auf der Basis von Phenolharzen, Epoxidharzen oder Polyestern, expandierte Microhohlkugeln mit Wandmaterial aus (Meth)acrylsäureester-Copolymeren, Polystyrol, Styrol(meth)acrylat-Copolymeren sowie insbesondere aus Polyvinylidenchlorid sowie Copolymeren des Vinylidenchlorids mit Acrylnitril und/oder (Meth)acrylsäureestern, keramische Hohlkugeln oder organische Leichtfüllstoffe natürlichen Ursprungs wie gemahlene Nussschalen, beispielsweise die Schalen von Cashewnüssen, Kokosnüssen oder Erdnussschalen sowie Korkmehl oder Kokspulver. Besonders bevorzugt werden dabei solche Leichtfüllstoffe auf der Basis von Mikrohohlkugeln, die in der ausgehärteten Zubereitung eine hohe Druckfestigkeit gewährleisten.

Weiterhin können die erfindungsgemäßen härtbaren Zubereitungen gängige weitere Hilfs- und Zusatzmittel wie beispielsweise Weichmacher, Rheologie-Hilfsmittel, Netzmittel, Haftvermittler, Alterungsschutzmittel, Stabilisatoren und/oder Farbpigmente enthalten.

Die beiden Zubereitungen der erfindungsgemäßen Zweikomponentenzusammensetzung müssen bis unmittelbar vor der Anwendung separat voneinander gelagert werden.

Dies kann vorzugsweise durch eine Konfektionierung in getrennten Behältern erfolgen. Die Entnahme der Zubereitung kann dann unmittelbar vor der Anwendung erfolgen. Dies kann beispielsweise durch manuelle Abmessung der benötigten Mengen aus Vorratsgefäßen, wie beispielsweise Fässern erfolgen.

Die Dosierung kann neben der rein manuellen Abmessung auch durch einfache Handdosiergeräte bis hin zu vollautomatisierten Systemen erfolgen. Derartige Systeme werden beispielsweise von der der Firma Loctite® vertrieben. Beispiele für derartige automatisierte Systeme sind volumetrische Doppel-Zahnradpumpen, Doppelpräzisionskolbendosierer, Doppelschneckenpumpendosierer oder Fasspumpensysteme.

In einer besonders bevorzugten Ausführungsform der vorliegenden Erfindung werden die erfindungsgemäßen Zubereitungen in entsprechenden Kartuschen, mit einem Volumenverhältnis von 1:1, 1:2 beziehungsweise 1:10 (Komponente mit Epoxyharzmischung : Komponente mit Thioetherverbindung) angeboten. Durch die Wahl derartiger Doppelkartuschen soll erfindungsgemäß eine gleich bleibende Dosierung und somit ein konstantes Mischungsverhältnis der beiden Komponenten zueinander sichergestellt werden. Die erfindungsgemäßen Kartuschen können bei der Anwendung mit Hilfe von einfachen Handdosiergeräten, aber auch mit pneumatischen und/oder vollständig automatisierten Systemen entleert werden.

In einer anderen Konfektionierungsform können die beiden Komponenten der erfindungsgemäßen Zusammensetzung coextrudiert werden und dementsprechend bis zur Anwendung unmittelbar nebeneinander vorliegend konfektioniert sein. Zum Zeitpunkt der Anwendung müssen dann die beiden Komponenten gründlich miteinander durchmischt werden. Dies kann beispielsweise durch Kneten des Coextrudates erfolgen.

Ein vierter Gegenstand der vorliegenden Erfindung ist ferner das ausgehärtete Produkt der erfindungsgemäßen Zweikomponentenzusammensetzung.

Ein fünfter Gegenstand der vorliegenden Erfindung ist Verfahren zum Verkleben von Werkstoffen, bei dem

a) gegebenenfalls die Oberflächen der zu verklebenden Werkstoffe gereinigt und/oder vorbehandelt werden,
b) eine Härterzusammensetzung, enthaltend mindestens eine erfindungsgemäße Thioetherverbindung, mit einer zweiten Zubereitung vermischt wird, die mindestens ein reaktives Harz enthält,
c) die resultierende Anwendungszubereitung auf mindestens eine der zu verklebenden Werkstoffoberflächen aufgetragen wird,
d) anschließend die zu verklebenden Werkstoffoberflächen zusammengefügt werden und
e) abschließend die aufgetragene Anwendungszubereitung bei Umgebungstemperaturen zwischen -10°C und 80°C, vorzugsweise zwischen 15°C und 30°C, ausgehärtet wird.

Auch an dieser Stelle sei bezüglich der bevorzugten Ausführungsformen explizit auf die Ausführungen im Rahmen der anderen Gegenstände der vorliegenden Erfindung verwiesen.

## Ausführungsbeispiele

*1 Herstellung der erfindungsgemäßen Thioverbindungen*

In einem 250ml 3-Halskolben wurden unter Stickstoffatmosphäre Capcure® 3-800 (278g/eq Thiol EW) vorgelegt, auf 70°C erhitzt und langsam 16,51g DMAPMA (Dimethylaminopropylmethacrylamid; M=170g/mol) zugetropft. Im Anschluss wurde für mindestens 2h bei 70°C gerührt.

| Probe | Capcure® 3-800 [g] | DMAPMA [g] | Thiol EW : M(DMAPMA) |
|---|---|---|---|
| A-1 | 90 | 16,51 | 1:0,3 |
| A-2 | 90 | 55,04 | 1:1 |
| A-3 | 90 | 27,52 | 1:0,5 |
| A-4 | 90 | 11,01 | 1:0,2 |

In allen Proben war der DMAPMA-Gehalt nach 2 Stunden Reaktionszeit <1,5 Gew.-% (bestimmt durch HPLC Analyse); nach 4 Stunden Reaktionszeit betrug der DMAPMA Gehalt nur noch <0,4Gew.-%.

Das ThiolEW wird in der Einheit [eq/g] angegeben und ist definiert als:

$$\text{ThiolEW [eq/g]} = 1/(\text{MercaptanZahl [meq/g]}/1000).$$

Die Mercaptan Zahl wird vom Produzenten bestimmt.

Herstellung der Klebstoffe

Es wurden die folgenden Zweikomponentenklebstoffe hergestellt, ausgehärtet und die Zugscherfestigkeiten ermittelt.

| Probe | Harzmischung | | Härter | Zusatz | LSS [N/mm$^2$] | Verarbeitungs -zeit [min] |
|---|---|---|---|---|---|---|
| K-1 | 2,38g<br>2,38g<br>1,19g | DEN®439<br>DER®732<br>Epikote® 1002 | 4,23g A-3 | -- | 13,53 | 4 |
| K-2 | 2,21g<br>2,44g<br>1,16g | DEN®439<br>DER®732<br>Epikote® 1002 | 4,23g A-1 | -- | 10,39 | 5 |
| K-3 | 2,14g<br>2,14g<br>0,58g | DEN®439<br>DER®732<br>Epikote® 1002 | 5,13g A-2 | -- | 12,29 | 3,5 |
| K-4 | 2,35g<br>2,35g<br>1,18g | DEN®439<br>DER®732<br>Epikote® 1002 | 4,12g A-2 | -- | 9,38 | 5 |
| K-5 | 2,30g<br>2,30g<br>1,15g | DEN®439<br>DER®732<br>Epikote® 1002 | 4,21g A-1 | 0,04g TETA (Triethylentetramin) | 15,42 | 4,5 |
| K-5 | 1,76g<br>1,76g<br>0,88g | DEN®439<br>DER®732<br>Epikote® 1002 | 3,24g A-1 | 2,06g Luzenac® 2<br>0,29g Cabosil® TS-720 | 20,00 | 4,5 |
| K-6 | 4,76g | DER® 331 | 5,24g A-1 | -- | 11,19 | 2 |

1.1 Verzeichnis der eingesetzten Rohstoffe

| | |
|---|---|
| Cabosil® TS-720 | Siliziumdioxid, Pyrogene amorphe Kieselsäure, Hersteller Cabot |
| Capcure® 3-800 | Mercaptanterminiertes flüssiges Polymer; Mercaptanzahl mind. 3,0 meg/g; Mercaptane equivalent weight 278 g/eq.; Herstellter Cognis |
| DEN® 439 | Reaktionsprodukt von Epichlorhydrin mit einem Phenol/Formaldehyd-Novolak; EEW 200g/eq; Epoxyfunktionalität ±3,8; Hersteller Dow |
| DER® 331 | Reaktionsprodukt von Bisphenol A mit Epichlorhydrin; EEW 187g/eq; Hersteller Dow |
| DER® 732 | Reaktionsprodukt von Epichlorhydrin mit Polypropylenglykol; EEW 320g/eq; Hersteller Dow |
| Epikote® 1002 | Reaktionsprodukt von Epichlorhydrin mit Bisphenol A; EEW 638g/eq; Hersteller Hexion; |

| | |
|---|---|
| Luzenac® 2 | natürliche Assoziation von Talk, Chlorite und Dolomite; Hersteller Luzenac Group |
| Versamine® EH-30 | 2,4,6-Tris(dimethylaminomethyl)phenol; 100% Aktivsubstanzgehalt; Hersteller Cognis |

1.2 Bestimmung der Zugscherfestigkeiten

Zur Bestimmung der Zugscherfestigkeiten wurden die Klebstoffe auf zwei sandgestrahlte, kaltgewälzte Stahlproben mit einer Überlappungsfläche von 2,5 $cm^2$ und einer Schichtdicke von 0,2mm aufgetragen, und die beiden Proben miteinander verklebt. Anschließend wurden die Proben für 7 Tage bei Raumtemperatur ausgehärtet. Nach dieser Zeit wurden die Zugscherfestigkeit des Klebstoffs gemäß DIN EN 1465 mit einer Geschwindigkeit von 15mm/min getestet. Die ermittelten Werte sind in der obigen Tabelle vermerkt.

Mit allen Harz(mischung)en ergaben die erfindungsgemäßen Thioverbindungen Verklebungen mit einer zufrieden stellenden Zugscherfestigkeit.

1.3 Bestimmung der Verarbeitungszeit

Für die Bestimmung der Verarbeitungszeit wurden 10g der jeweiligen Klebstoffmischung in eine Schale gegeben. In kurzen Abständen wird ein Holzspatel in die Klebstoffmischung getaucht (senkrecht zur Oberfläche der Klebstoffmischung) und wieder herausgezogen. Die Verarbeitungszeit ist der Zeitraum zwischen Mischen und Herausziehen des Holzspatels, wenn beim Herausziehen des Holzspatels keine Klebstofffäden mehr entstehen.

**Patentansprüche**

1. Verwendung einer Thioetherverbindung, erhältlich aus einem ersten Edukt, das mindestens zwei Thiolgruppen aufweist, und einem zweiten Edukt, das mindestens eine $\alpha,\beta$-ungesättige Amid-Gruppe sowie mindestens eine tertiäre Amin-Gruppe aufweist, zum Härten reaktiver Harze, die eine mittlere Funktionalität größer 1 aufweisen und ausgewählt sind aus Harzen auf Isocyanatbasis, Harzen auf Acrylat- und Methacrylatbasis, Anhydriden sowie Epoxid-basierten Systemen.

2. Verwendung nach Anspruch 1, **dadurch gekennzeichnet, dass** die Thioetherverbindung

   a. mindestens eine Thioethergruppe,
   b. gegebenenfalls eine oder mehrere Thiol-Gruppen,
   c. mindestens eine Amid-Gruppe und
   d. mindestens eine tertiäre Amin-Gruppe

   im Molekül aufweist, mit der Maßgabe, dass die Thioetherverbindung mindestens zwei Schwefel-haltige funtionelle Gruppen, ausgewählt aus Thioethergruppen und/oder Thiolgruppen, aufweist.

3. Verwendung nach Anspruch 2, **dadurch gekennzeichnet, dass** die Thioetherverbindung mindestens eine Gruppe der Formel (I)

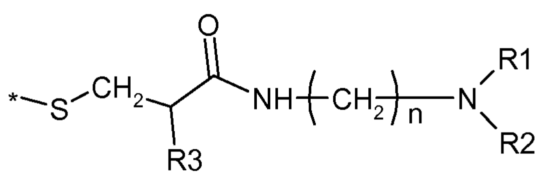

(I)

   trägt, wobei

   - R1 und R2 stehen unabhängig voneinander für eine $C_1$- bis $C_{20}$-Alkylgruppe,
   - R3 steht für ein Wasserstoffatom oder eine $C_1$- bis $C_4$-Alkylgruppe,
   - n steht für eine ganze Zahl von 1 bis 20,
   - mit der Maßgabe, dass die Gruppe der Formel (I) über ein Kohlenstoffatom an den Rest des Moleküls gebunden ist.

**4.** Verwendung nach Anspruch 3, **dadurch gekennzeichnet, dass** die Thioetherverbindung eine Verbindung der Formel (II)

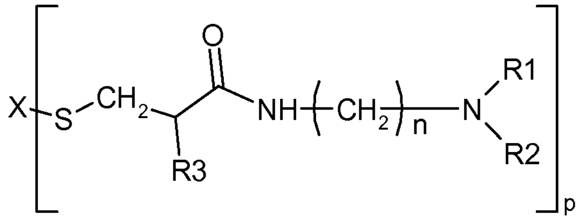

(II)

ist, wobei

- R1 und R2 stehen unabhängig voneinander für eine $C_1$- bis $C_{20}$-Alkylgruppe,
- R3 steht für ein Wasserstoffatom oder eine $C_1$- bis $C_4$-Alkylgruppe,
- n steht für eine ganze Zahl von 1 bis 20,
- p steht für eine rationale Zahl von 1,0 bis 5,0 und
- X steht für einen p-valenten Rest, der jeweils über ein Kohlenstoffatom an die funktionelle Gruppe der Formel (I) gebunden ist, und gegebenenfalls mindestens eine Thiol-Gruppe und/oder mindestens eine Thioether-Gruppe aufweist,
- mit der Maßgabe, dass X zwingend mindestens eine Thiol-Gruppe und/oder mindestens eine Thioether-Gruppe aufweist, wenn p=1 ist.

**5.** Verwendung nach Anspruch 4, **dadurch gekennzeichnet, dass**

- R1 und R2 stehen für eine Methylgruppe und/oder
- R3 steht für eine Methylgruppe und/oder
- n steht für die ganze Zahl 3 und/oder
- X steht für einen oligomeren oder polymeren Rest.

**6.** Verwendung einer Thioetherverbindung nach einem der Ansprüche 4 oder 5, **dadurch gekennzeichnet, dass** der Rest X Alkylendioleinheiten, insbesondere Ethylenglykoleinheiten und/oder Propylenglykoleinheiten und/oder Butandioleinheiten aufweist.

**7.** Verwendung nach einem der Ansprüche 1 bis 6, **dadurch gekennzeichnet, dass** das reaktive Harz ein Epoxidharz ist.

**8.** Härterzusammensetzung für reaktive Harze, die eine mittlere Funktionalität größer 1 aufweisen und ausgewählt sind aus Harzen auf Isocyanatbasis, Harzen auf Acrylat- und Methacrylatbasis, Anhydriden sowie Epoxid-basierten Systemen,
**dadurch gekennzeichnet, dass** sie mindestens eine Thioetherverbindung nach einem der Ansprüche 1 bis 7 sowie einen Beschleuniger enthält, der mindestens ein Stickstoffatom aufweist.

**9.** Zweikomponentige Zusammensetzung, deren erste Zubereitung mindestens ein reaktives Harz enthält, das eine mittlere Funktionalität größer 1 aufweist und ausgewählt ist aus Harzen auf Isocyanatbasis, Harzen auf Acrylat- und Methacrylatbasis, Anhydriden sowie Epoxid-basierten Systemen,
und deren zweite Zubereitung mindestens eine Thioetherverbindung nach einem der Ansprüche 1 bis 6 enthält.

**10.** Gehärtetes Produkt der zweikomponentigen Zusammensetzung nach Anspruch 9.

**11.** Verfahren zum Verkleben von Werkstoffen, **dadurch gekennzeichnet, dass**

a) gegebenenfalls die Oberflächen der zu verklebenden Werkstoffe gereinigt und/oder vorbehandelt werden,
b) die zwei Komponenten der Zweikomponentenzusammensetzung nach Anspruch 9 miteinander vermischt werden,
c) die resultierende Anwendungszubereitung auf mindestens eine der zu verklebenden Werkstoffoberflächen aufgetragen wird,

d) anschließend die zu verklebenden Werkstoffoberflächen zusammengefügt werden und
e) abschließend die aufgetragene Anwendungszubereitung bei Umgebungstemperaturen zwischen -10°C und 80°C, vorzugsweise zwischen 15°C und 30°C, ausgehärtet wird.

**Claims**

1. The use of a thioether compound, which can be obtained from a first starting material comprising at least two thiol groups and a second starting material comprising at least one a, β unsaturated amide group and at least one tertiary amine group, for curing reactive resins that have an average functionality of greater than 1 and are selected from resins based on isocyanate, resins based on acrylate and methacrylate, anhydrides and epoxy-based systems.

2. The use according to claim 1, **characterized in that** the thioether compound comprises

   a. at least one thioether group,
   b. optionally one or more thiol groups,
   c. at least one amide group and
   d. at least one tertiary amine group

   in the molecule, with the proviso that the thioether compound comprises at least two sulfur-containing functional groups selected from thioether groups and/or thiol groups.

3. The use according to claim 2, **characterized in that** the thioether compound carries at least one group of formula (I)

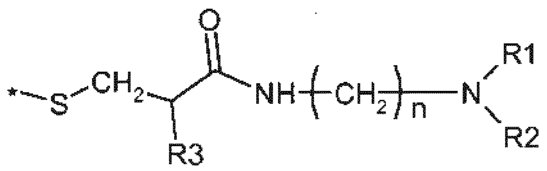

(I)

   where

   - R1 and R2 represent, independently of one another, a $C_1$ to $C_{20}$ alkyl group,
   - R3 represents a hydrogen atom or a $C_1$ to $C_4$ alkyl group,
   - n represents an integer from 1 to 20,
   - with the proviso that the group of formula (I) is bonded to the group of the molecule by a carbon atom.

4. The use according to claim 3, **characterized in that** the thioether compound is a compound of formula (II)

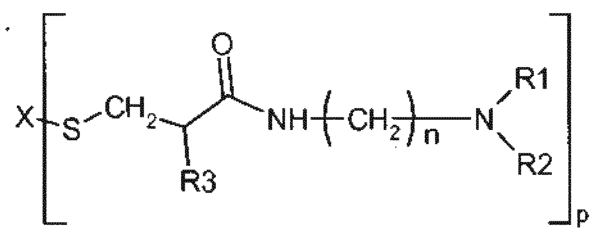

(II)

   where

   - R1 and R2 represent, independently of one another, a $C_1$ to $C_{20}$ alkyl group,
   - R3 represents a hydrogen atom or a $C_1$ to $C_4$ alkyl group,
   - n represents an integer from 1 to 20,
   - p represents a rational number from 1.0 to 5.0 and
   - X represents a p-valent group which is bonded to the functional group of formula (I) by one carbon atom in each case, and optionally comprises at least one thiol group and/or at least one thioether group,
   - with the proviso that X must contain at least one thiol group and/or at least one thioether group when p=1.

**5.** The use according to claim 4, **characterized in that**

- R1 and R2 represent a methyl group and/or
- R3 represents a methyl group and/or
- n represents the integer 3 and/or
- X represents an oligomeric or polymer group.

**6.** The use of a thioether compound according to one of claims 4 or 5, **characterized in that** the group X comprises alkylene diol units, in particular ethylene glycol units and/or propylene glycol units and/or butane diol units.

**7.** The use according to one of claims 1 to 6, **characterized in that** the reactive resin is an epoxy resin.

**8.** A curing agent composition for reactive resins that have an average functionality of greater than 1 and are selected from resins based on isocyanate, resins based on acrylate and methacrylate, anhydrides and epoxy-based systems, **characterized in that** it contains at least one thioether compound according to one of claims 1 to 7 and an accelerator that comprises at least one nitrogen atom.

**9.** A two-component composition of which the first preparation contains at least one reactive resin that has an average functionality of greater than 1 and is selected from resins based on isocyanate, resins based on acrylate and methacrylate, anhydrides and epoxy-based systems,
and of which the second preparation contains at least one thioether compound according to one of claims 1 to 6.

**10.** A cured product of the two-component composition according to claim 9.

**11.** A method for bonding materials, **characterized in that**

a) the surfaces of the materials to be bonded are optionally cleaned and/or pretreated,
b) the two components of the two-component composition according to claim 9 are mixed together,
c) the resulting preparation for use is applied to at least one of the material surfaces to be bonded,
d) the material surfaces to be bonded are then joined together and
e) the applied preparation for use is lastly cured at ambient temperatures of between -10°C and 80°C, preferably of between 15°C and 30°C.

## Revendications

**1.** Utilisation d'un composé thioéther obtenu à partir d'un premier produit de départ comprenant au minimum deux groupements thiol et d'un deuxième produit de départ comprenant au minimum un groupe amide a, β insaturé et au moins un groupe amine tertiaire pour le durcissement des résines réactives présentant une fonctionnalité moyenne supérieure à 1 et sélectionnées parmi des résines à base d'isocyanate, des résines à base d'acrylate et de méthacrylate, des anhydrides et des systèmes à base d'époxyde.

**2.** Utilisation selon la revendication 1, **caractérisée en ce que** le composé thioéther présente

a. au moins un groupement thiol,
b. un ou plusieurs groupements thiol, le cas échéant,
c. au moins un groupe amide et
d. au moins un groupe amine tertiaire

dans la molécule, à condition que le composé thioéther comprenne au moins deux groupes fonctionnels contenant du souffre sélectionnés parmi des groupes thioéther et/ou des groupes thiol.

**3.** Utilisation selon la revendication 2, **caractérisée en ce que** le composé thioéther présente au moins un groupe de formule (I)

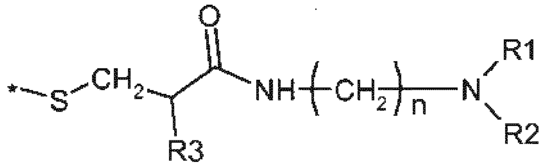

(I)

où

- R1 et R2 représentent indépendamment l'un de l'autre un groupe alkyle $C_1$-$C_{20}$,
- R3 représente un atome d'hydrogène ou un groupe alkyle $C_1$-$C_4$,
- n est un nombre entier compris entre 1 et 20,
- à condition que le groupe de formule (I) soit relié au reste de la molécule par un atome de carbone.

4. Utilisation selon la revendication 3, **caractérisée en ce que** le composé thioéther est un composé de formule (II)

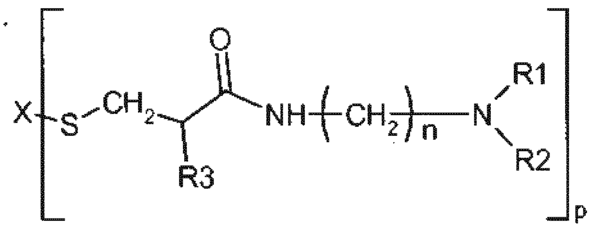

(II)

où

- R1 et R2 représentent indépendamment l'un de l'autre un groupe alkyle $C_1$-$C_{20}$,
- R3 représente un atome d'hydrogène ou un groupe alkyle $C_1$-$C_4$,
- n est un nombre entier compris entre 1 et 20,
- p est un nombre rationnel compris entre 1,0 et 5,0 et
- X représente un résidu p-valent relié au groupe fonctionnel de formule (I) par un atome de carbone et présentant au moins un groupe thiol, le cas échéant et/ou au moins un groupe thioéther,
- à condition que X présente obligatoirement au moins un groupe thiol et/ou au moins un groupe thioéther lorsque p = 1.

5. Utilisation selon la revendication 4, **caractérisée en ce que**

- R1 et R2 représentent un groupe méthyle et/ou
- R3 représente un groupe méthyle et/ou
- n représente le chiffre entier 3 et/ou
- X représente un résidu oligomère ou polymère.

6. Utilisation d'un composé thioéther conforme à l'une des revendications 4 ou 5, **caractérisé en ce que** le résidu X présente des alkylènediols, en particulier des éthylèneglycols et/ou des propylèneglycols et/ou des butanediols.

7. Utilisation selon l'une des revendications 1 à 6, **caractérisée en ce que** la résine réactive est une résine époxyde.

8. Composition d'agent durcisseur pour résine réactive présentant une fonctionnalité moyenne supérieure à 1 et sélectionnée parmi des résines à base d'isocyanate, des résines à base d'acrylate et de méthacrylate, des anhydrides et des systèmes à base d'époxyde, **caractérisée en ce qu'**elle contient au minimum un composé thioéther conforme à l'une des revendications 1 à 7 ainsi qu'un accélérateur présentant au minimum un atome d'azote.

9. Structure à deux composants dont la première préparation contient au moins une résine réactive présentant une fonctionnalité moyenne supérieure à 1 et sélectionnée parmi des résines à base d'isocyanate, des résines à base d'acrylate et de méthacrylate, des anhydrides et des systèmes à base d'époxyde, et dont la deuxième préparation comprend au moins un composé thioéther conforme à l'une des revendications 1 à 6.

**10.** Produit durci de la structure à deux composants selon la revendication 9.

**11.** Procédé de liaison de matières **caractérisé en ce que**

a) les surfaces de ces matières sont nettoyées et/ou font l'objet d'un traitement préalable, le cas échéant,
b) les deux composants de la structure sont mélangés selon la revendication 9,
c) la préparation d'application obtenue est appliquée sur au moins l'une des surfaces des matières à lier,
d) les surfaces des matières sont ensuite assemblées et
e) la préparation appliquée est ensuite durcie à une température ambiante comprise entre -10 et 80 °C, et de préférence entre 15 et 30 °C.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0370446 A2
- DE 19729875 A1
- EP 2006310 A2